(19) Europäisches Patentamt
European Patent Office
Office européen des brevets

(11) Publication number: **0 072 015**
**B1**

(12) **EUROPEAN PATENT SPECIFICATION**

(45) Date of publication of patent specification: **31.10.84**

(51) Int. Cl.$^3$: **C 07 C 125/065**

(21) Application number: **82107181.8**

(22) Date of filing: **09.08.82**

(54) **Method for purifying N-benzyloxycarbonyl aspartic acid.**

(30) Priority: **10.08.81 US 291548**

(43) Date of publication of application:
**16.02.83 Bulletin 83/07**

(45) Publication of the grant of the patent:
**31.10.84 Bulletin 84/44**

(84) Designated Contracting States:
**BE DE FR GB NL**

(56) References cited:

**CHEMICAL ABSTRACTS, vol. 68, no. 19, 6th May 1968, page 8452, no. 87512u, Columbus, Ohio, USA V. KALIS et al.: "Preparative chromatography in peptide chemistry. I. Purification of some carbobenzoxyamino acids and peptides by means of preparative adsorption chromatography"**

(73) Proprietor: **PPG INDUSTRIES, INC.**
**One PPG Place**
**Pittsburgh Pennsylvania 15272 (US)**

(72) Inventor: **Sathe, Avinash Trimbak**
**W-123 West Shore Drive**
**Mundelein Illinois 60060 (US)**

(74) Representative: **Hann, Michael, Dr. et al**
**Patentanwälte Dr. Michael Hann Dr. H.-G. Sternagel Marburger Strasse 38**
**D-6300 Giessen (DE)**

**EP 0 072 015 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

## Description

The lower alkyl esters of $\alpha$-L-aspartyl-L-phenylalanine that have 1 to 4 carbon atoms in the alkyl group have a taste closely resembling that of sugar and are, therefore, valuable sugar substitutes. See, for example, U.S. Patent 3,492,131. One such dipeptide, i.e., L-aspartyl-L-phenylalanine methyl ester (aspartame), is described as 100 to 200 times as sweet as sucrose. The afore-mentioned dipeptide esters are conveniently manufactured from the aspartic acid derivative wherein the amino function is protected by a benzyloxycarbonyl group, the $\beta$-carboxy function is protected by a benzyl ester group, and the $\alpha$-carboxy group is protected by a p-nitrophenyl ester group. The preparation of that aspartic acid derivative is described by S-Guttmann in Helv. Chim. Acta, 44, 721 (1961). Further reaction of that derivative with a phenylalanine ester, e.g., L-phenylalanine methyl ester, produces a protected dipeptide which when subjected to hydrogenolysis produces the lower alkyl ester, e.g., the methyl ester of L-aspartyl-L-phenylalanine. The aforesaid aspartic acid derivative can be prepared from N-benzyloxycarbonyl-L-aspartic acid.

The use of the aforementioned dipeptide sweetening agents in edible materials requires that aspartic acid derivatives utilized as precursers in the synthesis of the dipeptide sweetening agent be as pure as possible. Thus, it is most desirable that such precursers be substantially free of by-products formed during their chemical synthesis.

N-benzyloxycarbonyl aspartic acid, a precursor, of the aforesaid aspartic acid derivative, is commonly prepared by reacting a water soluble alkali metal salt of L-aspartic acid with benzyl chloroformate. In the course of that reaction, a small quantity of the alkali metal salt of N-benzyloxycarbonyl aspartic acid reacts with the L-aspartic acid salt reactant to form a minor contaminating amount of the by-product adduct, N-benzyloxycarbonyl aspartyl aspartic acid.

It has now been discovered that this by-product impurity, i.e., N-benzyloxycarbonyl aspartyl aspartic acid, can be removed from the N-benzyloxycarbonyl aspartic acid product by alkaline hydrolysis of the N-benzyloxycarbonyl aspartic acid product. In particular, it has been discovered that N-benzyloxycarbonyl aspartic acid containing less than 0.2, preferably less than 0.1, weight percent of the N-benzyloxycarbonyl aspartyl aspartic acid by-product can be prepared by digesting alkali metal salt of the by-product containing aspartic acid product at a pH of at least about 10 and at temperatures of from 50°C. to 90°C. for a time sufficient to hydrolyze substantially all of the N-benzyloxycarbonyl aspartyl aspartic acid. A preferred temperature range is from 65°C. to 85°C. and suitable digestion periods are from 0.5 to 12, e.g., 1 to 4, hours.

*Detailed Description*

The reaction of aspartic acid with benzyl chloroformate has been described in the literature. See, for example, the article, "Concerning a Universal Process for the Synthesis of Peptides" by Max Bergmann et al, Berichte d.D. Chem. Gesellschaft, Volume 65, pages 1192—1201, 1197 (1932) and Houben-Weyl, Volume XV/1, pages 321 (1974). The reaction is also described in Example 1 of U.S. Patent 3,808,190.

In a typical process for the preparation of N-benzyloxycarbonyl aspartic acid (hereinafter Z-Asp), benzyl chloroformate is charged gradually to a reaction vessel containing an aqueous solution of a dialkali metal salt of L-aspartic acid under controlled conditions of pH and temperature. The pH of the aqueous solution can range between about 7 and 14 during addition of the benzyl chloroformate. Preferably, the pH is maintained at between about 10 and 12, more preferably between 10.75 and 11.75, e.g., between 11.5 and 11.75 in order to reduce the amount of N-benzyloxycarbonyl aspartyl aspartic acid (hereinafter Z-Asp Asp) by-product produced and obtain adequate yields of the desired Z-Asp product. At a pH significantly greater than 11.75, e.g., between 12 and 13.5, significant hydrolysis of the benzyl chloroformate reactant to benzyl alcohol occurs and the Z-Asp product is found to contain more than trace amounts of impurities that are less polar than Z-Asp, as determined by thin layer chromatography (TLC). The yield of Z-Asp is also reduced when the reaction is conducted at a high pH, i.e., a pH of 12 to 13.5. When the pH of the aqueous solution is significantly less than 10.75, e.g., between about 7 and 9, significant quantities, e.g., from about 0.5 to about 16 weight percent (basis Z-Asp) of Z-Asp Asp as well as impurities less polar and more polar than Z-Asp, as determined by TLC, are found in the Z-Asp product.

The Z-Asp product purified in accordance with the present invention contains minor contaminating amounts of Z-Asp Asp. While the herein described process is applicable to Z-Asp containing as much as 16 weight percent Z-Asp Asp, the process will usually be employed with Z-Asp product containing much less Z-Asp Asp since procedures for minimizing the amount of Z-Asp Asp product initially will typically be employed to maximize the yield of Z-Asp. Typically, the Z-Asp will contain less than 5, e.g., less than 1 or 2 weight percent, of the Z-Asp Asp, Z-Asp containing from 0.2 or 0.3 to 5, e.g., 0.2 or 0.3 to 1 or 2, weight percent Z-Asp Asp will usually be treated in accordance with the herein described method.

As described hereinabove, an aqueous solution of an alkali metal salt of the Z-Asp Asp — containing Z-Asp product is maintained at a pH of at least 10 for a time sufficient to hydrolyze

substantially all of the Z-Asp Asp impurity. Preferably, the pH is maintained at at least 12, more preferably at at least 13, e.g., 13 to 13.5 (the maximum being a pH of 14). The particular pH chosen should be that hydroxyl ion concentration which provides an adequate rate of hydrolysis for commercial applications. While, it has been discovered unexpectedly that the Z-Asp product is substantially unaffected by the herein described treatment, the use of excessive amounts of alkaline reagent is economically undesirable.

The aforesaid pH of the Z-Asp aqueous solution to be treated is obtained by the addition of a water soluble, inorganic alkaline reagent to an aqueous slurry of the contaminated Z-Asp product or to an aqueous solution of an alkali metal salt of such Z-Asp product having a pH less than 10. Preferably, the alkaline reagent used is an alkali metal hydroxides, e.g., sodium hydroxide or potassium hydroxide.

The amount of alkaline reagent added to the Z-Asp slurry or aqueous solution is that amount required to bring to and maintain a solution of the material to be treated at the desired pH for the period of treatment. The concentration of the alkaline reagent used is not critical. In the case of sodium or potassium hydroxide, it is common to utilize between a 20 and 50 weight percent solution thereof.

The above-described hydrolysis reaction is conducted at temperatures between 50°C. and 90°C., preferably, between 65°C. and 85°C. The rate at which the hydrolysis reaction proceeds is a direct function of the temperature used. However, as the temperature approaches the upper limit within the aforesaid range, the greater the tendency for decomposition of the Z-Asp product. At temperatures of from 20°C. to 50°C., the rate of hydrolysis is slow. At temperatures of less than about 20°C., (room temperature), the reaction proceeds at commercially impractical rates.

The length of time for which the aqueous solution of the alkali metal salt of Z-Asp is maintained at the desired pH will vary depending on the temperature and pH used. Generally, the higher the temperature used, the shorter the time required for complete alkaline hydrolysis of the Z-Asp Asp by-product. Commonly, time periods of from 0.5 to 12 hours, more usually from 1 to 4 hours are used. Such periods are usually sufficient to hydrolyze substantially all of the Z-Asp Asp by-product, i.e., produce Z-Asp containing less than 0.2 weight percent Z-Asp Asp.

Purification of Z-Asp containing minor amounts of Z-Asp Asp by alkaline hydrolysis can produce a Z-Asp product substantially free of Z-Asp Asp. By substantially free is meant that the Z-Asp recovered contains less than 0.2 weight percent Z-Asp Asp. More preferably, the recovered Z-Asp contains less than 0.1, most preferably less than 0.05, weight percent of Z-Asp Asp.

The Z-Asp product produced in accordance with the present process is substantially pure, i.e., it is at least 97 percent (on a weight basis) Z-Asp. Often the product is better than 98 or 99 percent pure. In addition to any remaining unhydrolyzed Z-Asp Asp and other organic impurities, the Z-Asp product can contain small amounts (not more than about one percent) of alkali metal salt, e.g., sodium or potassium chloride, and water. Other organic impurities, i.e., substances that are less polar and more polar than Z-Asp, as determined by thin layer chromatography (TLC), usually represent less than an estimated 0.5, usually less than 0.2 weight percent of the product. The amount of alkali metal salt remaining in the Z-Asp product is controlled, in part, by the thoroughness with which the product is washed, e.g., with water, following its recovery.

The alkaline hydrolysis treatment described herein can be applied to a Z-Asp product or to a metal e.g., alkali metal, salt thereof, e.g., as an added step to the process for preparing Z-Asp. In the former embodiment, the Z-Asp is slurried in water and the acid product titrated with alkali, e.g., sodium hydroxide, to form the soluble alkali metal salt of the acid. Treatment of the resulting aqueous solution then proceeds as described herein. The amount of water used is not critical; but, preferably should be sufficient only to serve as a solvent for the salt and form an easily handled reaction mixture. Since Z-Asp is soluble to a degree in water, the more water used, the more product lost when the Z-Asp product is later separated.

In the latter embodiment, following the reaction sequence which produces the Z-Asp product (usually as a soluble alkali metal salt), the pH of the reaction medium is raised to the desired level for alkaline hydrolysis by the addition of an alkaline reagent, e.g., an alkali metal hydroxide such as sodium hydroxide, and the process of the present invention conducted.

Following alkaline hydrolysis, the Z-Asp product is recovered by acidification of the reaction mixture with a mineral acid, e.g., hydrochloric or sulfuric aid, to a pH at which the Z-Asp crystallizes from the aqueous solution. The crystalline product is recovered by any conventional solid-liquid separation technique, e.g., filtration, centrifugation, etc. The wet cake recovered can be washed one or more times with water, usually cold water, and the washed product dried, e.g., at 40°C. in a suitable oven.

The aspartic acid used in the preparation of Z-Asp is available commercially as the D(—), L(+) or DL isomers. Any of the aforementioned isomers can be used. For use in preparing sweetening agents, the L(+) stereoisomer is preferred (the DL isomer mixture being less preferred) for the reason that the L-stereoisomer appears to provide the sweetening properties desired.

Benzyl chloroformate is available commercially. It is added slowly and with appropriate

agitation in substantially stoichiometric amounts to the reaction mixture in order to avoid the presence of excessive or localized quantities of unreacted benzyl chloroformate within the reaction mixture. Sufficient agitation to promote intimate contact between the reactants should be used. In a preferred embodiment, the rate at which benzyl chloroformate is introduced into the reaction mixture is controlled so that the mole ratio of unreacted benzyl chloroformate to the aspartic acid salt therein is not greater than 0.2, preferably not greater than 0.15 and most preferably not greater than 0.1.

The addition of benzyl chloroformate to the reaction mixture at a rate which results in significant quantities of unreacted benzyl chloroformate in the reaction mixture, e.g. by adding the benzyl chloroformate all at once, results in hydrolysis of more than a minor portion of the benzyl chloroformate. The resulting benzyl alcohol forms esters with acidic materials, i.e., carboxylic acid containing materials, thay may be present within the reaction mixture. These esters are believed to be the less polar impurities found by TLC in the Z-Asp product. It has been found that the addition gradually and uniformly of benzyl chloroformate to the reactor over a period of at least about 2 hours, e.g., 2 to 3 hours, is sufficient to maintain the mole ratio of unreacted benzyl chloroformate to aspartic acid salt in the reaction mixture at less than the above described level of 0.2 and to control the rate at which heat is generated by the reaction.

The following is an exemplification of the preparation of Z-Asp. Benzyl chloroformate is added to an aqueous solution of an alkali metal salt of aspartic acid. The aforementioned aqueous solution can be prepared by adding alkali metal hydroxide to an aqueous slurry of the acid. As used in the present specification and claims, the term "alkali metal" intended to mean and include sodium and potassium. In order to obtain an aqueous aspartic acid alkali metal salt solution having the pH desired for the reaction, is is necessary to utilize an alkaline reagent that will provide an aqueous solution at that pH. Sodium hydroxide and potassium hydroxide are examples of such reagents.

In preparing the aspartic acid salt solution, it is convenient to slurry the aspartic acid in water and subsequently add, e.g., by titration, the alkali metal hydroxide to the slurry until the desired pH is reached. However, it is possible to add the aspartic acid to an aqueous solution of alkali metal hydroxide. The aspartic acid is thereby neutralized to form its alkali metal salt, which is water soluble. Slightly more than a stoichiometric amount of alkali metal hydroxide, i.e., sodium or potassium hydroxide, (2 moles of hydroxide per mole of aspartic acid) are required to prepare the aforesaid solution.

The amount of water used to prepare the aspartic acid solution is not critical; however, excessive amounts of water are economically disadvantageous. Therefore, only that amount of water which provides a reaction mixture that can be easily handled should be used. For example, a ratio of 0.55 liters of water per mole of aspartic acid has been found to be suitable. The Z-Asp product is soluble to a degree in water, the degree of solubility being a direct function of the temperature of the solution. Thus, the more water used to prepare the aspartic acid solution, the more product lost when the aqueous phase of the reaction mixture is separated from the product.

The overall reaction for the preparation of the disodium salt of Z-Asp (N-benzyloxycarbonyl aspartic acid) can be depicted by the following balance equation:

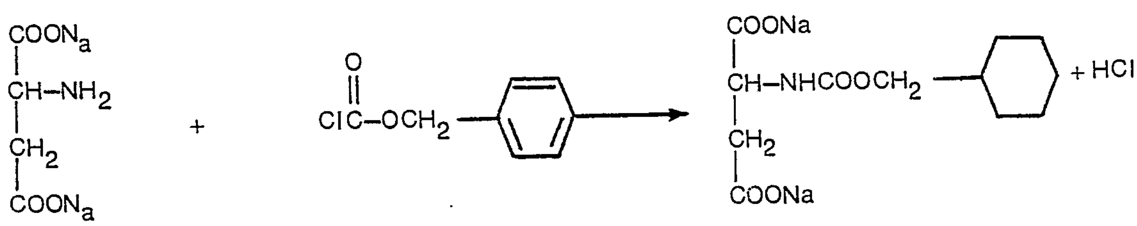

N-Benzyloxycarbonyl
aspartic acid,
disodium salt.

Reaction of the sodium salt of N-benzyloxycarbonyl aspartic acid with a further mole of the aspartic acid sodium salt yields (following acidification) the undesired impurity, N-benzyloxycarbonyl aspartyl aspartic acid (Z-Asp Asp),

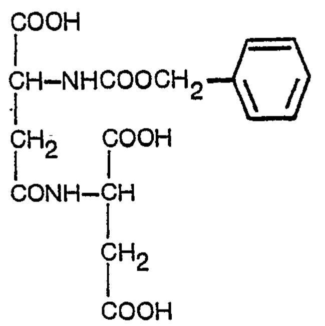

N-Benzyloxycarbonyl aspartyl aspartic acid

Although the aforesaid formula for Z-Asp Asp has been shown as the $\beta$-amide it is believed that any Z-Asp Asp impurity formed is likely to be present as both the $\alpha$- and $\beta$-amides.

After completing the addition of benzyl chloroformate to the reaction mixture, agitation and addition of alkali metal hydroxide are continued until complete reaction of the benzyl chloroformate occurs. About 0.5 to 1 hour (depending on the rate of benzyl chloroformate addition) is required for the reaction to reach completion, which is indicated by stabilization of the pH of the reaction mixture. Stabilization of the pH is indicated when no additional alkali metal hydroxide is required to be added to the reaction mixture to maintain the pH thereof at the level used to conduct the reaction. Thereafter, the reaction mixture is acidified with cooling to a pH of between about 1.5 and about 2.5 to convert the Z-Asp alkali metal salt product to the free acid. Examples of acids that can be used include hydrochloric acid and sulfuric acid. Preferably, hydrochloric acid is used as the acid so as not to introduce a further anionic species into the reaction medium. Sufficient cooling is provided to compensate for the heat of neutralization.

The Z-Asp product (free acid) in the acidified reaction mixture crystallizes from the reaction mixture and is separated from its mother liquor by conventional separating means, e.g., by means of a filter or centrifuge. The recovered white granular Z-Asp product is washed with water to remove alkali metal salt and dried at temperatures less than its decomposition temperature. Washing of the recovered wet Z-Asp product with an equal weight of water (a larger quantity of water can be used if desired) and drying in vacuum or in a circulating air oven at 40°C. have been found to be satisfactory to obtain as essentially pure product.

The present process is more particularly described in the following examples which are intended as illustrative only since numerous modifications and variations therein will be apparent to those skilled in the art.

Example 1

731 grams of 96.4% N-benzyloxycarbonyl aspartic acid, which was prepared by reaction of the sodium salt of L-aspartic acid with benzylchloroformate at a pH of about 11.3, and which contained about 0.44 weight percent of N-benzyloxycarbonyl aspartyl aspartic acid was dissolved in 2000 milliliters of tap water at room temperature. To this solution were slowly added 300 milliliters of 50% sodium hydroxide. The temperature and pH of the solution rose from about 22°C. and 2.19 to 55°C. and 11.3 respectively. The alkaline solution was then heated to 85°C. During the first 20 minutes of heating, the pH of the alkaline solution dropped to about 9.9. The pH was raised by the addition of 20 milliliters of additional 50% sodium hydroxide to the alkaline solution and the solution stirred for 30 minutes until the pH appeared to stabilize at 10.42.

The alkaline solution was held at 85°C. for one hour and then cooled to 8°C. The pH of the cooled solution was 12.17. To this cooled solution were added 450 milliliters of concentrated hydrochloric acid. The acidified solution was stirred for 90 minutes, at which point the solution had a pH of 2.28. The precipitate which formed was filtered and the filter cake washed with 300 milliliters of ice tap water. The washed cake was dried in a 40°C. vacuum oven and 647 grams (88%) recovery) of dried product obtained. Analysis of the dried product by liquid chromotography indicated that it was about 97.2% N-benzyloxylcarbonyl aspartic acid which contained less than 0.1 weight % of N-benzyloxycarbonyl aspartyl aspartic acid.

Example II

To a one liter round bottom flask containing 400 milliliters of tap water were added 146.2 grams of about 80% N-benzyloxycarbonyl aspartic acid (prepared in the manner described in Example I) containing about 2.78 weight percent of N-benzyloxycarbonyl aspartyl acid and about 17.9 weight percent water. Analysis for Z-Asp and Z-Asp Asp was by liquid chromotography; analysis for water was by the Karl Fischer method — hence totals of greater than 100% are possible. To this solution, 51.7 milliliters of 50% sodium hydroxide were added slowly. The pH of the aspartic acid solution rose from an initial value of 2.19 to 11.3 while the temperature increased from 22°C. to about 39°C. during addition of 48.5 milliliters of the sodium hydroxide. At this point, the solution was heated to 85°C. over 40 minutes during which time the pH dropped to 9.72. The remaining 3.2 milliliters of sodium hydroxide were added and the solution stirred for about $2\frac{1}{2}$ hours while maintaining the temperature at 85°C. The final pH was 12.30.

The alkaline solution was acidified to a pH of 1.5 by the addition of 72.4 milliliters of concentrated hydrochloric acid. The acidified solution was stirred overnight at room temperature and 18.0 milliliter of concentrated hydrochoric acid added. The resulting acidified solution (pH of 0.95) was cooled to 8°C. and stirred for 4.5 hours. The precipitate which formed was filtered and the filter cake washed with 80 milliliters of ice tap water. The wet cake was vacuum dried for 24 hours at 60°C. to yield 99.3 grams of dried material. Analysis of the dried product showed it to be 95.5% N-benzyloxycarbonyl aspartic acid which contained 1.04 weight percent N-benzyloxycarbonyl aspartyl aspartic acid.

Example III

To a multi-necked round bottom flask fitted with a thermometer, mechanical stirrer, addition funnels, condenser and pH probe were added 310 milliliters of water and 66.5 grams of L-aspartic acid at room temperature. The resulting slurry was titrated with 50% aqueous sodium hydroxide using the pH probe and a pH meter to a pH of 10.3. While maintaining the resulting solution at about 20°C., 87.8 grams of benzyl chloroformate (98.5%) was slowly added to the flask over 2 hours. The pH of the solution in the flask was maintained at about 10 during addition of the benzyl chloroformate by the simultaneous addition of 25% aqueous sodium hydroxide.

Following addition of the benzyl chloroformate, the reaction mixture was stirred for about thirty minutes at which time the pH appeared to have stabilized at about 10.1. A portion of the reaction mixture was withdrawn from the flask, cooled to 10°C., and acidified with concentrated hydrochloric acid until a pH of 1.5 was attained. The resulting crystalline product was recovered by filtration and the filter cake washed with an equal weight of water. The washed cake was dried in a 40°C. oven overnight. Analysis of the dried product by liquid chromotography indicated that the product was 97.6% N-benzyloxycarbonyl aspartic acid which contained 0.37 weight percent of N-benzyloxycarbonyl aspartyl aspartic acid.

To the room temperature reaction mixture remaining in the reaction flask was added 12.5 grams of 25% sodium hydroxide with stirring over four hours until the pH was brought up to 13.25. The mixture in the flask was acidified over one hour with concentrated hydrochloric acid and the resulting crystalline precipitate recovered by filtration. The filter cake was washed with an equal weight of water and dried in a 40°C. oven. The dried product (88 grams) was analyzed by liquid chromatography, which indicated that it was substantially all N-benzyloxycarbonyl aspartic acid with less than 0.05 weight percent N-benzyloxycarbonyl aspartyl aspartic acid. The total yield of N-benzyloxycarbonyl aspartic acid was 88%, based on the L-aspartic acid.

The data of Examples I—III show that N-benzyloxycarbonyl aspartic acid can be treated at a high alkaline pH to remove by hydrolysis minor amounts of N-benzyloxycarbonyl aspartyl aspartic acid contained therein. The data of Example II shows that a longer digestion period was required to reduce further the Z-Asp Asp content of the Z-Asp product. As described, digestion for $2\frac{1}{2}$ hours reduced the Z-Asp Asp content by almost 2 percent.

Although the present process has been described with reference to specific details of certain embodiments thereof, it is not intended that such details should be regarded as limitations upon the scope of the invention except as and to the extent that they are included in the accompanying claims.

## Claims

1. A process for purifying N-benzyloxycarbonyl aspartic acid containing minor contaminating amounts of N-benzyloxycarbonyl aspartyl aspartic acid, which comprises digesting an aqueous solution of an alkali metal salt of said N-benzylcarbonyl aspartic acid at a pH of at least about 10 and at temperatures of from 50°C. to 90°C. for a time sufficient to hydrolyze substantially all of the N-benzyloxycarbonyl aspartyl aspartic acid.

2. The process of claim 1 wherein the contaminating amount of N-benzyloxycarbonyl aspartyl aspartic acid is from 0.2 to 5 weight percent.

3. The process of claim 1 wherein the alkali metal salt is the sodium or potassium salt.

4. The process of claim 1 wherein the time of digestion is from 0.5 to 12 hours.

5. The process of claim 3 wherein the time of digestion is from 1 to 4 hours.

6. The process of claim 1 wherein the digested solution is acidified with hydrochloric or sulfuric acid, thereby to produce N-benzyloxycarbonyl aspartic acid substantially free of N-benzyloxycarbonyl aspartyl aspartic acid.

7. The process of claim 6 wherein the digested N-benzyloxycarbonyl aspartic acid contains less than 0.1 weight percent N-benzyloxycarbonyl aspartyl aspartic acid.

8. In the process for preparing N-benzyloxycarbonyl aspartic acid wherein an aqueous alkaline solution of alkali metal salt of aspartic acid is reacted with benzyl chloroformate and the resulting reaction mixture is acidified to crystallize N-benzyloxycarbonyl aspartic acid, the improvement which comprises digesting the reaction mixture prior to acidification at a pH of at least 10 and at a temperature of from 50°C. to 90°C. for from about 0.5 to 12 hours, and acidifying the digested reaction mixture to crystallize N-benzyloxycarbonyl aspartic acid therefrom, thereby to produce N-benzyloxycarbonyl aspartic acid substantially free of N-benzyloxycarbonyl aspartyl aspartic acid.

9. The process of claim 8 wherein the period of digestion is from 1 to 4 hrs.

10. The process of claim 8 wherein hydrochloric acid is used to acidify the digested reaction mixture.

**Revendications**

1. Procédé pour purifier de l'acide N-benzyloxycarbonyl aspartique contenant des quantités mineures d'acide N-benzyloxycarbonyl apartyl aspartique en tant qu'impureté caractérisé en ce qu'il comprend la digestion d'une solution aqueuse d'un sel de métal alcalin de cet acide N-benzylcarbonyl aspartique, à un pH d'au moins 10 environ et à des températures de 50°C à 90°C pendant un temps suffisant pour hydrolyser la quasi-totalité de l'acide N-benzyloxycarbonyl aspartyl aspartique.

2. Procédé selon la revendication 1, dans lequel la quantité d'acide N-benzyloxycarbonyl aspartyl aspartique en tant qu'impureté se situe entre, 0,2 et 5% en poids.

3. Procédé selon la revendication 1, dans lequel le sel de métal alcalin est le sel de sodium ou de potassium.

4. Procédé selon la revendication 1, dans lequel le temps de digestion est de 0,5 à 12 heures.

5. Procédé selon la revendication 3, dans lequel le temps de digestion est de 1 à 4 heures.

6. Procédé selon la revendication 1, dans lequel la solution est, après digestion, acidifiée par de l'acide chlorhydrique ou sulfurique de manière à produire de l'acide N-benzyloxycarbonyl aspartique quasiment exempt d'acide N-benzyloxycarbonyl aspartyl aspartique.

7. Procédé selon la revendication 6, dans lequel l'acide N-benzyloxycarbonyl aspartique contient, après digestion, moins de 0,1% en poids d'acide N-benzyloxycarbonyl aspartyl aspartique.

8. Perfectionnement au procédé de préparation de l'acide N-benzyloxycarbonyl aspartique, selon lequel on fait réagir une solution alcaline aqueuse d'un sel de métal alcalin de l'acide aspartique avec du chloroformiate de benzyle et l'on acidifie le mélange réactionnel résultant pour cristalliser l'acide N-benzyloxycarbonyl aspartique, ledit perfectionnement étant caractérisé en ce qu'il consiste à soumettre le mélange réactionnel, avant acidification, à une digestion à un pH d'au moins 10 et à une température de 50°C à 90°C pendant environ 0,5 à 12 heures, et à acidifier le mélange réactionnel résultant de la digestion pour en cristalliser l'acide N-benzyloxycarbonyl aspartique, afin de produire de l'acide N-benzyloxycarbonyl aspartique quasiment exempt d'acide N-benzyloxycarbonyl aspartyl aspartique.

9. Procédé selon la revendication 8, dans lequel la période de digestion est de 1 à 4 heures.

10. Procédé selon la revendication 8, dans lequel on utilise de l'acide chlorhydrique pour acidifier le mélange réactionnel résultant de la digestion.

**Patentansprüche**

1. Ein Verfharen zum Reinigen von N-Benzyloxycarbonylasparaginsäure, die geringe verunreinigende Mengen von Benzyloxycarbonyl-asparaginyl-asparaginsäure enthält, dadurch gekennzeichnet, dass man eine wässrige Lösung eines Alkalisalzes dieser N-Benzylcarbonylasparaginsäure bei einem pH von mindestens etwa 10 und einer Temperatur von 50 bis 90°C für eine ausreichende Zeit digeriert, um im wesentlichen die gesamte N-Benzyloxycarbonyl-asparaginyl-asparaginsäure zu hydrolysieren.

2. Das Verfahren von Anspruch 1, dadurch gekennzeichnet, dass die verunreinigende Menge von N-Benzyloxycarbonyl-asparaginyl-asparaginsäure 0.2 bis 5 Gew% beträgt.

3. Das Verfahren nach Anspruch 1, dadurch gekennzeichnet, dass das Alkalisalz das Natrium- oder Kalium-salz ist.

4. Das Verfahren nach Anspruch 1, dadurch gekennzeichnet, dass die Digerierungszeit 0.5 bis 12 Stunden beträgt.

5. Das Verfahren nach Anspruch 3, dadurch gekennzeichnet, dass die Digerierungszeit 1 bis 4 Stunden beträgt.

6. Das Verfahren nach Anspruch 1, dadurch gekennzeichnet, dass die digerierte Lösung mit Chlorwasserstoffsäure oder Schwefelsäure angesäuert wird, um eine N-benzyloxycarbonylasparaginsäure herzustellen, die im wesentlichen frei von N-Benzyloxycarbonylasparaginyl-asparaginsäure ist.

7. Das Verfahren nach Anspruch 6, dadurch gekennzeichnet, dass die digerierte N-Benzyloxycarbonylasparaginsäure weniger als, 0,1 Gew% N-Benzyloxycarbonyl-asparaginylasparaginsäure enthält.

8. In dem Verfahren zur Herstellung von N-Benzyloxycarbonylasparaginsäure, bei dem eine wässrige alkalische Lösung eines Alkalisalzes der Asparaginsäure mit Benzylchlorformiat umgesetzt wird und die erhaltene Reaktionsmischung angesäuert wird, um N-Benzyloxycarbonylasparaginsäure auskristallisieren zu lassen, die Verbesserung, die dadurch gekennzeichnet ist, dass man die Reaktionsmischung vor dem Ansäuern bei einem pH von mindestens 10 und einer Temperatur von 50 bis 90°C für etwa 0.5 bis 12 Stunden digeriert und die digerierte Reaktionsmischung ansäuert, um aus ihr N-Benzyloxycarbonylasparaginsäure auskristallisieren zu lassen, wobei man N-Benzyloxycarbonylasparaginsäure erhält, die im wesentlichen frei von N-Benzyloxycarbonylasparaginyl-asparaginsäure ist.

9. Das Verfahren nach Anspruch 8, dadurch gekennzeichnet, dass der Zeitraum für die Digerierung 1 bis 4 Stunden beträgt.

10. Das Verfahren nach Anspruch 8, dadurch gekennzeichnet, dass zum Ansäuern der digerierten Reaktionsmischung Chlorwasserstoffsäure verwendet wird.